# EUROPÄISCHE PATENTSCHRIFT

(11) **EP 2 287 151 B1**
(45) Veröffentlichungstag und Bekanntmachung des Hinweises auf die Patenterteilung: **25.06.2014**
(21) Anmeldenummer: 10008259.3
(22) Anmeldetag: 07.08.2010
(51) Int. Cl.: C07C 273/18, C08G 18/78, C08G 18/32, C09D 175/04

(54) **Verfahren zur Herstellung von Polyisocyanaten mit Biuretstruktur**
Method for producing polyisocyanates with biuret structure
Procédé destiné à la fabrication de polyisocyanates dotés d'une structure de biuret

(30) Priorität: 21.08.2009 DE 102009038463
(43) Veröffentlichungstag der Anmeldung: 23.02.2011
(73) Patentinhaber: Bayer Intellectual Property GmbH, 40789 Monheim (DE)
(72) Erfinder: Brahm, Martin, Dr., 51519 Odenthal (DE); Mager, Dieter, 51373 Leverkusen (DE); Fellhölter, Andre, Dr., 51427 Bergisch Gladbach (DE); Halpaap, Reinhard, Dr., 51519 Odenthal (DE)
(74) Vertreter: BIP Patents

(56) Entgegenhaltungen:
- EP-A1- 1 158 013
- WO-A1-2008/110492
- LAAS H J ET AL: "ZUR SYNTHESE ALIPHATISCHER POLYISOCYANATE-LACKPOLYISOCYANATE MIT BIURET-, ISOCYANURAT- ODER URETDIONSTRUKTUR. ÖTHE SYNTHESIS OF ALIPHATIC POLYISOCYANATES CONTAINING BIURET, ISOCYANURATE OR URETDIONE BACKBONES FOR USE IN COATINGS", JOURNAL FUER PRAKTISCHE CHEMIE, WILEY VCH, WEINHEIM, Bd. 336, Nr. 3, 1. Januar 1994 (1994-01-01), Seiten 185-200, XP000441642, ISSN: 1436-9966, DOI: DOI:10.1002/PRAC.19943360302

## Beschreibung

Die vorliegende Erfindung betrifft ein verbessertes Verfahren zur Herstellung von Polyisocyanaten mit Biuretstruktur durch kontinuierliche Umsetzung von überschüssigen Mengen an organischen Diisocyanaten mit ausschließlich aliphatisch und/oder cycloaliphatisch gebundenen Isocyanatgruppen mit organischen Diaminen mit ausschließlich aliphatisch und/oder cycloaliphatisch gebundenen primären Aminogruppen bei erhöhten Temperaturen durch 2-stufige Zugabe der Isocyanatkomponente. So hergestellte Polyisocyanate zeichnen sich durch hohe Stabilität aus, sind mit großer Raumzeitausbeute herstellbar und weisen einen niedrigen Gehalt an Nebenprodukten auf. Bei gegebener Produktionsanlage sind auf einfache Weise verfahrenstechnische Varianten möglich um Biuretpolyisocyanate mit angestrebten Zielgrößen (Viskosität, NCO-Gehalt) zu erhalten.

Die Herstellung von aliphatischen Polyisocyanaten mit Biuretstrukturen ist seit 1958 bekannt (DE-A 1101394). Weitere mögliche Herstellverfahren sind in einem Übersichtsartikel (Laas et al., J. prakt. Chem. 336, 1994, 185-200) beschrieben, die Vor- und Nachteile der jeweiligen Verfahren diskutiert.

Man unterscheidet prinzipiell zwei Verfahrensgruppen: zum einen die sogenannten Wasser-verfahren, bei denen die Diisocyanate mit unterschüssigen Mengen an Wasser zu Harnstoffen und diese nachfolgend mit überschüssigen Mengen an Diisocyanaten zu Biureten umgesetzt werden, zum anderen die sogenannten Diisocyanat/Diamin-Verfahren, bei denen aus Isocyanat und unterschüssiger Menge an Amin unmittelbar Harnstoff und nachfolgend wiederum mit Überschuss an Diisocyanat Biuret hergestellt wird. Für beide Verfahren sind, wie im oben zitierten Übersichtsartikel (Laas et al.) ausgeführt, zahlreiche Varianten ausgearbeitet und beschrieben worden. Bei den beschriebenen Verfahren wird überwiegend mit Hexamethylendiisocyanat (HDI) und gegebenenfalls Hexamethylendiamin (HDA) zur Herstellung der technisch bedeutsamsten HDI-Biurete gearbeitet, wobei die zunächst erhaltenen in überschüssigem Diisocyanat gelöst vorliegenden Biurete durch Destillation und/oder Extraktion von überschüssigem Diisocyanat befreit und als monomerenarme Biuret-Polyisocyanate isoliert werden.

Nach Wasserverfahren hergestellte Biuret-Polyisocyanate zeichnen sich in der Regel durch eine gute Monomerenstabilität, d. h. Stabilität gegenüber teilweiser Rückspaltung in freie Diisocyanate, gute Verdünnbarkeiten, d. h. Stabilität verdünnter Lösungen bezüglich Trübungen und Ausfällungen bei Feuchteeinwirkung, sowie hervorragender Farbzahlen aufgrund der relativ milden Reaktionsbedingungen bei der Herstellung aus. Bei den Biuretisierungsreaktionen nach Wasserverfahren wird jedoch Prinzip-bedingt stets ein Teil der im Ausgangsgemisch enthaltenen Isocyanatgruppen durch Reaktion mit einem Biuretisierungsmittel intermediär in Aminogruppen umgewandelt. Da die so verbrauchten Isocyanatgruppen ursprünglich einmal durch Phosgenierung von Aminogruppen hergestellt wurden, erscheint diese Vorgehensweise aufwendig und wenig ökonomisch. Zudem entstehen bei diesen bekannten Verfahren gasförmige oder flüssige Nebenprodukte, wie zum Beispiel Kohlendioxid, Kohlenmonoxid, Kohlenoxidsulfid, Olefine oder Nitrile, die mit Ausnahme der beim Pivalinsäure-/Wasser-Verfahren anfallenden Anhydride nicht recyclisierbar sind und entsorgt werden müssen.

Bei den ausgearbeiteten Diisocyanat/Diamin-Verfahren zeigt sich der Vorteil der ökonomischen Herstellung mit keiner oder nur geringer Nebenproduktbildung, es werden keine durch Phosgenierung aus Aminogruppen hergestellte Isocyanatgruppen wieder in Aminogruppen, nachfolgend Harnstoff- und Biuretgruppen, zurückverwandelt. Diese Verfahren sind ebenfalls zu einem hohen Qualitätsstand ständig weiterentwickelt worden wie z. B. in der EP-A 277353 beschrieben. Eine verminderte Monomeren- und Verdünnungstabilität konnte durch weitere Optimierungen weiter verbessert werden, wie in EP-B 1158013 beschrieben.

Wie in der vorliegenden Literatur beschrieben, wird HDI auf Temperaturen von ca. 230 - 250 °C vorerhitzt und in einer Mischkammer mit HDA zur Reaktion gebracht. Hierbei steigt die Temperatur weiter auf Werte von typischerweise 270 - 280 °C. Danach wird die Temperatur stufenweise möglichst rasch auf z.B 180 °C abgekühlt. Um eine unnötig große Vorschädigung des temperaturempfindlichen HDI's zu vermeiden, wird das Edukt in möglichst kurzer Zeit aufgeheizt und überhitzt. Prinzipiell ist es jedoch nicht möglich, die Temperaturschädigung völlig auszuschließen.

Um eine schnelle Reaktion und Biuretbildung zu gewährleisten, werden spezielle Mischkammer/Düsensysteme zur optimalen und schnellen Durchmischung der Isocyanat- und Aminkomponente eingesetzt. Aus strömungstechnischen Gründen sind diese Systeme auf einen engen Mengenstrombereich ausgelegt und somit in der Variation begrenzt. Bei Laständerungen sowie bei Produktumstellung wird dieser optimale Bereich verlassen. Die nichtideale Mischung führt zu einer verzögerten und schlechteren Biuretbildung und größeren Harnstoffkristallen mit der Folge, dass sich die Gesamtreaktionszeit verlängert und Nebenreaktionen zunehmen.

Aus Umweltgesichtspunkten führt die Erhitzung der großen Mengenströme an HDI und HDA auf hohe Temperaturen mit der ebenso notwendigen anschließenden raschen Abkühlung zu einem hohen Energieverbrauch mit großem Kohlendioxid-Emissionspotential.

Die Aufgabe der vorliegenden Erfindung war es daher nach dem wirtschaftlich günstigen Diisocyanat/Diamin-Verfahren Polyisocyanate mit Biuretstruktur zu erhalten, ohne das Diisocyanat zu lange und bei zu hoher Temperatur zu belasten und die Herstellung bei Last- oder Produktumstellung immer mit dem von den Mischelementen optimalen Durchmischungsbedingungen zu betreiben. Die Aufgabe der Erfindung war es insbesondere auch den hohen Energieverbrauch zur Erhitzung des Diisocyanatstroms gegenüber dem bislang praktizierten Verfahren abzusenken, ohne auf die erwiesenen Vorteile des Diisocyanat/Diamin-Verfahrens zu verzichten.

Wie jetzt überraschend gefunden wurde, ist es möglich, in einem kontinuierlichen Verfahren hochwertige Polyisocyanate mit Biuretstruktur auf Basis von organischen Isocyanaten mit ausschließlich aliphatisch und/oder cycloaliphatisch gebundenen Isocyanatgruppen mit organischen Aminen mit ausschließlich aliphatisch und/oder cycloaliphatisch gebundenen primären Aminogruppen bei gegenüber dem Stand der Technik verminderter Temperaturbelastung sowie bei Last- und Produktwechsel ohne Verlassen der optimalen Durchmischungsbedingungen unter reduziertem Energieeinsatz zu erhalten indem die Umsetzung wie nachfolgend beschrieben zweistufig durchgeführt wird.

Gegenstand der Erfindung ist somit ein Verfahren zur kontinuierlichen Herstellung von Polyisocyanaten mit Biuretstruktur durch kontinuierliche Umsetzung von überschüssigen Mengen an organischen Isocyanaten enthaltend ausschließlich aliphatisch und/oder cycloaliphatisch gebundene Isocyanatgruppen (A) mit organischen Aminen enthaltend ausschließlich aliphatisch und/oder cycloaliphatisch gebundene primäre Aminogruppen (B), indem man
a) eine erste Menge der Isocyanatkomponente A mit der Aminkomponente B vermischt, wobei die Temperatur oberhalb von 170 °C gehalten wird, und
b) eine zweite Menge der Isocyanatkomponente A mit einer Temperatur von 20 bis 250 °C dem Strom des aus Schritt a) resultierenden Reaktionsgemisches zudosiert,
wobei das Verhältnis der pro Zeiteinheit in Stufe a) zu b) eingespeisten Gewichtsteile der Isocyanatkomponente A 1:9 bis 9:1 beträgt, und die Anzahl der in a) und b) insgesamt pro Zeiteinheit eingespeisten NCO-Gruppen zur Zahl der pro Zeiteinheit eingespeisten NH₂-Gruppen mindestens 4:1 beträgt.

Bei den hohen Reaktionstemperaturen von oberhalb 170 ° erfolgt in einer Gleichgewichtsreaktion eine unmittelbare Reaktion zwischen Isocyanat- und Amingruppen zu Harnstoff- und Biuretstrukturen. Je nach eingestellter Reaktionstemperatur und verwendetem Isocyanatüberschuss liegt das Gleichgewicht hierbei fast vollständig auf der Seite des Harnstoffs und Biurets.

Die Gesamtmenge an Diisocyanat, Teilmenge aus Stufe a) und Teilmenge aus Stufe b), zur Menge des in Stufe a) umgesetzten Amins wird so gewählt, wie bislang beim einstufigen Diisocyanat-/Diamin-Verfahren üblich um ein Biuretpolyisocyanat mit den angestrebten Kenndaten zu erhalten.

Überraschenderweise führt das während des Mischvorgangs in Stufe a) gegenüber dem einstufigen Verfahren deutlich reduzierte Verhältnis von Isocyanat- zur Aminkomponente nicht zu einem anderen (üblicherweise höherviskosen) Produkt mit geringerem NCO-Gehalt. Dies ist um so überraschender, da gerade die Isocyanat/Aminreaktion bei den hohen Reaktionstemperaturen augenblicklich stattfindet, was sich durch die starke Exothermie direkt bei der Mischung zeigt, die ein Aufheizen des Produktstroms um ca. 20 bis 80 °C bewirkt.

Es war demnach ganz und gar nicht zu erwarten, dass die erfindungsgemäße 2-stufige Verfahrensweise beim Diisocyanat/Diamin-Verfahren zu den gleichen Produkten führt wie eine direkte einstufige Dosierung der Aminkomponete zu der gesamten Isocyanatkomponente.

Bei der zweistufigen Verfahrensweise reicht das Aufheizen des 1. Isocyanatstromes auf eine im Vergleich zur einstufigen Verfahrensweise geringere Temperatur aus, um die nötige Misch- und Reaktionstemperatur zu erlangen. Unabhängig von Last- und Produktwechsel kann der eigentliche Mischvorgang von Isocyanat- und Aminkomponente immer mit gleichen Mengenströmen an Isocyanat und Amin erfolgen. Der Rest der Isocyantkomponente, die zweite Teilmenge, wird nachträglich zudosiert, ohne dass sich das hergestellte Fertigprodukt in seinen Kenndaten ändert. Da gegenüber der einstufigen Fahrweise im erfindungsgemäßen Schritt a) die freiwerdende Reaktionsenthalpie einen geringeren Massestrom aufheizt, wird die erforderliche Reaktionstemperatur mit vergleichsweise niedriger Menge an vorgeheizter Isocyanatkomponente A erreicht. Durch die im erfindungsgemäßen Fall üblicherweise im Schritt b) während der Dosierung nochmalig verminderte Vorheiztemperatur der Isocyantkomponente A ergibt sich eine weitere deutliche Reduzierung des Energiebedarfs. Das Isocyanat wird insgesamt weniger thermisch belastet. Dies führt zur Verringerung der Nebenproduktbildung.

Ausgangsmaterialien für das erfindungsgemäße Verfahren sind organische Diisocyanate mit ausschließlich aliphatisch und/oder cycloaliphatisch gebundenen Isocyanatgruppen eines unter 300 liegenden Molekulargewichts. Beispiele derartiger Diisocyanate sind 1,4-Diisocyanatobutan, 1,6-Diisocyanatohexan (Hexamethylendiisocyanat, HDI), 1,6-Diisocyanato-2,2,4-trimethylhexan und/oder 1,6-Diisocyanato-2,4,4-trimethylhexan, 1,4-und/oder 1,5-Diisocyanatohexan, 2,6-Diisocyanatohexansäureethylester, 1,9-Diisocyanatononan, 1,12-Diisocyanatododecan, 1,4-Diisocyanatocyclohexan, 2,4- und/oder 2,6-Diisocyanato-1-methylcyclohexan, 1-Isocyanato-3,3,5-trimethyl-5-isocyanatomethylcyclohexan (Isophorondiisocyanat, IPDI), 1,3- und/oder 1,4-Bis(isocyanatomethyl)cyclohexan, 2,4'- und/oder 4,4'-Diisocyanatodicyclohexylmethan oder 6-Isocyanatohexansäure-2-isocyanatoethylester. Beliebige Gemische derartiger Diisocyanate können ebenfalls verwendet werden. 1,6-Diisocyanatohexan (HDI) ist bevorzugt.

Weitere Ausgangsmaterialien für das erfindungsgemäße Verfahren sind organische Diamine mit ausschließlich aliphatisch und/oder cycloaliphatisch gebundenen primären Aminogruppen. Sie haben ein Molekulargewicht unter 300. Beispiele sind 1,2-Diaminoethan, 1,2-Diaminopropan, 1,3-Diaminopropan, 1,4-Diaminobutan, 1,6-Diaminohexan (Hexamethylendiamin, HDA), 1,6-Diamino-2,2,4-trimethylhexan und/oder 1,6-Diamino-2,4,4-trimethylhexan, 1,4- und/oder 1,5-Diaminohexan, 2,4- und/oder 2,6-Diamino-1-methylcyclohexan, 1-Amino-3,3,5-trimethyl-5-aminomethylcyclohexan (Isophorondiamin, IPDA), 1,3- und/oder 1,4-Bis(aminomethyl)cyclohexan oder 2,4'- und/oder 4,4'-Diaminodicyclohexylmethan. Beliebige Gemische derartiger Diamine können ebenfalls eingesetzt werden. 1,6-Diaminohexan (HDA) ist bevorzugt.

Prinzipiell ist zur Reduzierung der Funktionalität auch die Mitverwendung von Monoaminen möglich. Die Mitverwendung von Monoaminen erfolgt gegebenenfalls in Mengen bis zu 30 Äquivalent-%, vorzugsweise bis zu 20 Äquivalent-%, besonders bevorzugt bis zu 10 Äquivalent-% bezogen auf die Summe der Äquivalente aus Diaminen und Monoaminen. Ganz besonders bevorzugt werden jedoch keine Monoamine mitverwendet. Falls Monoamine zum Einsatz kommen, werden aliphatische und/oder cycloaliphatische primäre Amine eines unterhalb oder maximal bei 185 liegenden Molekulargewichts verwendet wie beispielsweise Methylamin, Ethylamin, Propylamin, Butylamin, Pentylamin, Hexylamin, Octylamin, Dodecylamin, Cyclohexylamin, wobei die unterschiedlichen Isomere zum Einsatz kommen können und die Amine auch in beliebigen Abmischungen verwendet werden können.

Bei der Durchführung des erfindungsgemäßen Verfahrens werden in Summe (Teilmenge aus Stufe a) und Teilmenge aus Stufe b)) die genannten Ausgangsisocyanate und Diamine in solchen Mengenverhältnissen zur Umsetzung gebracht, die einem Äquivalentverhältnis von Isocyanatgruppen zu Aminogruppen von mindestens 4:1, vorzugsweise von 8:1 bis 60:1 und besonders bevorzugt von 16:1 bis 50:1 entsprechen, wobei die primären Aminogruppen als monofunktionelle Gruppen in die Berechnung eingehen. Ganz besonders bevorzugt ist ein Äquivalentverhältnis von Isocyanatgruppen zu Aminogruppen von 18:1 bis 25:1. D.h. die Anzahl der in a) und b) insgesamt pro Zeiteinheit eingespeisten NCO-Gruppen zur Zahl der pro Zeiteinheit eingespeisten NH₂-Gruppen beträgt mindestens 4:1, vorzugsweise 8:1 bis 60:1, besonders bevorzugt 16:1 bis 50:1 und ganz besonders bevorzugt 18:1 bis 25:1.

Die Reaktion kann beim erfindungsgemäßen Verfahren katalysiert und unkatalysiert erfolgen. Wird das Verfahren katalysiert durchgeführt, so werden als Katalysatoren, wie in EP-B 1158013 beschrieben, bevorzugt beliebige Säuren, vorzugsweise Protonensäuren, mit einem pK_{S}-Wert < 10 eingesetzt. Bevorzugte saure Katalysatoren sind Phosphorsäure bzw. Phosphorsäureester, wie z. B. Methylphosphat, Ethylphosphat, n-Butylphosphat, n-Hexylphosphat, 2-Ethylhexylphosphat, Isooctylphosphat, n-Dodecylphosphat, Dimethylphosphat, Diethylphosphat, Di-n-propylphosphat, Di-n-butylphosphat, Di-n-amylphosphat, Diisoamylphosphat, Di-n-decylphosphat, Diphenylphosphat oder Dibenzylphosphat, Sulfonsäuren, wie z. B. Methansulfonsäure, Ethansulfonsäure, Propansulfonsäure, 2- bzw. 4-Toluolsulfonsäure oder Naphthalin-1-sulfonsäure, oder auch Mono- und Dicarbonsäuren, wie z.B. Ameisensäure, Essigsäure, Propionsäure, Buttersäure, Pivalinsäure, Stearinsäure, Cyclohexancarbonsäure, Oxalsäure, Malonsäure, Bersteinsäure, Adipinsäure, Benzoesäure oder Phthalsäure. Besonders bevorzugt sind Dialkylphosphate der genannten Art. Ganz besonders bevorzugter Katalysator ist Di-n-butylphosphat.

Diese Säuren kommen beim erfindungsgemäßen Verfahren in Mengen von 0 bis 1,0 Gew.-%, vorzugsweise von 0,02 bis 0,5 Gew.-% und ganz besonders bevorzugt von 0,05 bis 0,5 Gew.-%, bezogen auf die Gesamtmenge an eingesetzten Ausgangsdiisocyanaten und Diaminen zum Einsatz. Die Säuren können gelöst in einem geeigneten Lösungsmittel zugegeben werden. Vorzugsweise werden die Säuren in Substanz zugegeben.

Die katalysierte ist gegenüber der unkatalysierten Verfahrensweise bevorzugt.

Das erfindungsgemäße Verfahren wird vorzugsweise lösemittelfrei durchgeführt. Durchaus möglich ist jedoch auch die Mitverwendung geeigneter, unter den Reaktionsbedingungen inerter Lösungsmittel. Geeignet sind zum Beispiel Hexan, Ethylacetat, Butylacetat, 1-Methoxypropyl-2-acetat, Propylenglykoldiacetat, 2-Butanon, 4-Methyl-2-pentanon, Cyclohexanon, Toluol, Xylol, höher substituierte Aromaten, wie sie beispielsweise unter den Bezeichnungen Solventnaphtha^{®}, Solvesso^{®}, Isopar^{®}, Nappar^{®} (Deutsche EXXON CHEMICAL GmbH, Köln) und Shellsol^{®} (Deutsche Shell Chemie GmbH, Eschborn) im Handel sind, oder Trialkylphosphate, wie z. B. Trimethylphosphat, sowie beliebige Gemische solcher Lösemittel. Die Mitverwendung inerter Lösungsmittel ist nicht bevorzugt und muß falls überhaupt bezüglich Mengen und Siedepunkten unter den Reaktionsbedingungen einsetzbar sein.

Zur Durchführung des erfindungsgemäßen Verfahrens werden die Ausgangsmaterialien in der ersten Stufe sofort nach ihrer Durchmischung bei einer Temperatur von oberhalb 170°C, vorzugsweise von 200°C bis 320 °C, insbesondere von 230 bis 320°C miteinander zur Reaktion gebracht. Hierzu wird der erste Teilstrom der Isocyanatkomponente A (Stufe a)), von dem pro Zeiteinheit 10 bis 90 Gewichtsteile, vorzugsweise 40 bis 80 Gewichtsteile und besonders bevorzugt 50 bis 75 Gewichtsteile von A dem Aminstrom B zugeführt werden, auf Temperaturen oberhalb von 150 °C, vorzugsweise mindestens 180 °C, besonders bevorzugt 180 bis 240 °C und ganz besonders bevorzugt 200 bis 240 °C vorgeheizt. Vorzugsweise liegt bei der Dosierung a) ein Überschuss an Isocyanatgruppen der Isocyanatkomponente A gegenüber den Amingruppen der Aminkomponente B vor. Im Falle der Verwendung eines hohen Isocyanatüberschusses erübrigt sich dann oftmals ein Vorerhitzen der Diamine, im allgemeinen werden jedoch auch diese auf ca. 50 bis 200°C vorerhitzt. In der Regel kann davon ausgegangen werden, dass sich das Reaktionsgemisch auch in Abwesenheit einer Beheizung des Mischgefäßes unmittelbar nach seiner Herstellung durch Vermischen der Ausgangsmaterialien wegen der starken Wärmetönung der spontan ablaufenden Reaktion auf eine Temperatur erhitzt, die ca. 20 bis 80°C oberhalb der Temperatur liegt, die aufgrund des Aufheizens der Ausgangsmaterialien ohne Einbeziehung der Wärmetönung erwartet werden kann und die dann den oben angegebenen Reaktionstemperaturbereichen entspricht.

Die in jedem Fall erforderliche Aufheizung und Überhitzung der Isocyanate muss wegen der bekannten Temperaturempfindlichkeit dieser Verbindungen innerhalb eines möglichst kurzen Zeitraums bewerkstelligt werden. Vorzugsweise erfolgt nach einer ersten Aufheizung der Isocyanate deren Überhitzung auf Reaktionseingangstemperatur innerhalb eines Zeitraums von weniger als 30 Sekunden. Dies gelingt durch Verwendung von entsprechenden Wärmeaustauschaggregaten des Standes der Technik. Die Wärmetauscher können z.B. als Röhren-, Bündel- oder Plattenwärmeaustauscher ausgelegt sein. Mikrowärmetauscher und mikroprofilierte Wärmetauscher (MiProWa) sind ebenfalls einsetzbar. Sie können mit einem flüssigen Heizmedium, mit gespanntem Dampf oder mit direkter elektrischer Heizung betrieben werden. Besonders bevorzugt ist die Verwendung von solchen Wärmetauschern, die den Aufheizvorgang der Ausgangsdiisocyanate innerhalb eines Zeitraums von weniger als 3 Sekunden gestatten.

Die kontinuierlichen Ströme der Reaktionspartner werden nach der beschriebenen Vorheizung in einer Mischkammer vereinigt. Beliebige statische oder dynamische Aggregate des Standes der Technik können zum Einsatz kommen. Ebenfalls einsetzbar sind einfache Mischkammern ausgeführt als ein einfaches Reaktionsrohr ohne jegliche Einbauten, an dessen einem Ende die Reaktionskomponenten im Gleichstrom eingebracht werden. Durchmischungsaggregate mit auf den Durchfluss optimierter Geometrie sind bevorzugt, um die Produktqualität und eine hohe Ausbeute zu gewährleisten.

Die Eintrittsstellen der Komponenten und Austrittsstellen des Reaktionsgemisches sind vorzugsweise in Form von Lochblenden oder Düsen ausgebildet, damit die Zudosierung unter Überdruck erfolgen kann. Dadurch kann gewährleistet werden, dass das Reaktionsgemisch nicht in die Zuleitungen von Diisocyanat und Diamin gelangen kann. Hierfür werden die Querschnitte so gewählt, dass sich auf den Zuleitungen jeweils ein Druck von 1,5 bis 100 bar, vorzugsweise von 1,5 bis 40 bar aufbaut.

Bei einer zusätzlichen Dosierung von Säuren erfolgt diese zweckmäßigerweise im Bereich der Mischkammer, vorzugsweise in die Isocyanatkomponente unmittelbar vor der Dosierung des Amins. Zur Eindosierung der Säuren können übliche Pumpen des Standes der Technik, wie z. B. Kolben- oder Kolbenmembranpumpen, verwendet werden. Es ist lediglich erforderlich, dass der Eindosierdruck höher als der Mischkammerdruck ist.

Nach Durchlaufen der Mischkammer und einer sich ggf. anschließenden Verweilzeitstrecke wird der 2. Teilstrom der Isocyanatkomponente A (Stufe b)) mit 90 bis 10 Teilen vorzugsweise 60 bis 20 Teilen und besonders bevorzugt 50 bis 25 Teilen pro Zeiteinheit zur Reaktionsmischung zudosiert.

Das Verhältnis der pro Zeiteinheit in Stufe a) zu b) eingespeisten Gewichtsteile der Isocyanatkomponente A beträgt 1:9 bis 9:1, bevorzugt 4:6 bis 8:2, besonders bevorzugt 5:5 bis 7,5: 2,5.

Die Zusammensetzung des Isocyanatstroms in Stufe b) kann gleich oder verschieden von der des Isocyanatstroms in Stufe a) sein. Im Gegensatz zum Isocyanatstrom aus a) werden hier üblicherweise keine katalytisch wirkende Zusätze zugegeben. Prinzipiell kann auch die Isocyanatkomponente des Isocyanatstroms in b) durch vorhergehende Vorreaktionen wie Urethanisierung, Allophanatisierung, oder Trimerisierung modifiziert sein. Dies ist jedoch weniger bevorzugt. Vorzugsweise handelt es sich beim Isocyanatstrom in b) um das gleiche Isocyanat wie im Isocyanatstrom in a). Besonders bevorzugt wird sowohl als Isocyanatstrom in a) als auch als Isocyanatstrom in b) HDI eingesetzt.

An die Geometrie der Dosierstrecke sind keine großen Anforderungen gestellt. Eine einfache Rohrzuführung (T-Stück) oder eine Düse oder Lochscheibe sind ausreichend. Bei turbulenter Strömung kann auf Mischeinbauten im Reaktionsrohr verzichtet werden.

Die Temperatur des 2. Isocyanat-Teilstroms in Stufe b) beträgt 20 bis 250°C, besonders bevorzugt 20 bis 230°C und ganz besonders bevorzugt 100 bis 220 °C. Die Temperatur des 2. Isocyanat-Teilstroms in Stufe b) kann die gleiche Temperatur wie die des 1. Isocyanat-Teilstroms in Stufe a) aufweisen, sofern die Temperatur des ersten Teilstromes maximal 250°C beträgt. Bevorzugt liegt die Temperatur des zweiten Teilstromes jedoch unterhalb der Temperatur des 1. Teilstroms.

Mit dem 2. Teilstrom sollte vorzugsweise die Gesamttemperatur des sich einstellenden Gemisches unter der der ersten Mischung aus Teilstrom 1 und Aminstrom liegen. Die Temperatur des 2. Teilstroms sollte so gewählt werden, dass die Gesamttemperatur nach der Dosierung kleiner 255 °C vorzugsweise kleiner 235 °C und besonders bevorzugt zwischen 235 und 170 °C liegt.

Nach der Zudosierung des 2. Isocyanatstroms (Stufe b)) sollte in Abhängigkeit der sich ergebenden Temperatur die Verweilzeit der heißen Reaktionsmischung bis zu der sich anschließenden Aufarbeitsstufe möglichst kurz gehalten werden. Bei Temperaturen von > 235 - 255 °C liegt die optimale Verweilzeit im Bereich von 1 sec bis 10 min, bei einer Temperatur von 200 - 235 °C liegt die Verweilzeit im Bereich zwischen 30 sec und 120 min, bei Temperaturen von 170 - < 200 °C liegt die Verweilzeit bei 10 min bis zu 8 h. Die optimale Verweilzeit muss für jede Anlage ermittelt werden.

Innerhalb der gesamten Verweilzeitstrecke erfolgt zumeist kontinuierlich eine Abkühlung durch geeignete Wärmeaustauscher und Rohrleitungen. Die Temperatur wird hierbei kontinuierlich auf einen Bereich von 80 bis 220°C, vorzugsweise 120 bis 200°C abgekühlt, wobei die oben genannten Temperatur- / Verweilzeit-Bereiche zur Nachtemperung erfüllt werden. Innerhalb der Verweilzeitstrecke können sich auch beispielsweise kaskadenförmig angeordnete Reaktoren befinden, um eine optimale mittlere Verweilzeit zu gewährleisten. Bei einer von 220 °C auf ca. 180 °C abgestuften Verweilzeitstrecke liegt die optimale Verweilzeit bei ca. 30 min.

Im Anschluß an die thermische Nachbehandlung liegt als Reaktionsprodukt eine Lösung von Biuretgruppen-aufweisenden Polyisocyanaten in überschüssigem Ausgangsisocyanat und gegebenenfalls mitverwendeten Lösungsmitteln vor, falls diese nicht bereits während der Umsetzung abdestilliert worden sind. Das vorliegende Gemisch wird anschließend im allgemeinen durch Destillation im Hochvakuum, bevorzugt im Dünnschichtverdampfer, beispielsweise bei einer Temperatur von 100 bis 200°C, vorzugsweise von 120 bis 180°C, von flüchtigen Bestandteilen (überschüssigen monomeren Diisocyanaten und gegebenenfalls mitverwendeten Lösungsmitteln) befreit. Als Verdampfer können dabei beliebige geeignete Ausführungsformen zum Einsatz kommen, wie Entspannungsverdampfer, Umlaufverdampfer, Fallrohrverdampfer, Fallfilmverdampfer, Dünnschichtverdampfer, Kurzwegverdampfer, die gegebenenfalls stufenweise parallel oder hintereinander geschaltet sein können.

In einer weiteren Ausführungsform des erfindungsgemäßen Verfahrens werden die genannten flüchtigen Bestandteile durch Extraktion mit geeigneten gegenüber Isocyanatgruppen inerten Lösungsmitteln, beispielsweise aliphatischen oder cycloaliphatischen Kohlenwasserstoffen wie Pentan, Hexan, Heptan, Cyclopentan oder Cyclohexan vom Reaktionsprodukt abgetrennt. Gegebenenfalls können zur Extraktion auch andere geeignete Extraktionsmittel wie beispielsweise Fluorkohlenwasserstoffe verwendet werden.

Nach der Aufarbeitung durch Extraktion oder Destillation erhält man hochwertige Polyisocyanate mit Biuretstruktur, die einen Gehalt an überschüssigem Ausgangsdiisocyanat von maximal 0,5 Gew.-%, vorzugsweise von maximal 0,3 Gew.-%, aufweisen.

Die nach dem erfindungsgemäßen Verfahren hergestellten, Biuretgruppen aufweisenden Polyisocyanate, insbesondere jene, die unter ausschließlicher Verwendung von 1,6-Diisocyanatohexan und 1,6-Diaminohexan als Ausgangsmaterialien hergestellt worden sind, stellen wertvolle Ausgangsmaterialien für die Herstellung von Zweikomponenten Polyurethanlacken dar. Die erfindungsgemäß hergestellten Produkte weisen ebenso wie die bisher bekannten Biuretpolyisocyanate des Standes der Technik gute Farbzahlen und vergleichsweise niedrige Viskositäten auf.

Der Anteil an Nebenreaktionen (z. B. der erhaltene Uretonimingehalt) ist jedoch geringer als beim einstufigen Diisocyanat-/Diamin-Verfahren, was die Monomerenstabilität des Polyisocyanats begünstigt.

### Beispiele

In den nachfolgenden Beispielen beziehen sich alle Prozentangaben auf Gewichtsprozente, Teile entsprechen Gewichtsteilen.

Die Ermittlung des NCO-Gehaltes der in den Beispielen und Vergleichsbeispielen beschriebenen Produkte erfolgte durch Titration gemäß DIN 53 185.

Die dynamischen Viskositäten wurden bei 23°C mit einem Viskosimeter Physica MCR 51, Fa. Anton Paar, Graz, AT bestimmt. Durch Messungen bei unterschiedlichen Schergeschwindigkeiten wurde sichergestellt, dass das Fließverhalten der beschriebenen erfindungsgemäß hergestellten Polyisocyanate wie auch das der Vergleichsprodukte dem Newtonscher Flüssigkeiten entspricht. Die Angabe der Schergeschwindigkeit kann deshalb entfallen.

### Beispiel 1

In einer Versuchsapparatur zur kontinuierlichen Herstellung von Biuret-Polyisocyanaten wurden 4600 Teile Hexamethylendiisocyanat (HDI) pro Stunde bei 214°C kontinuierlich durch eine Reaktionsmischkammer geleitet. In die HDI-Zuleitung kurz vor der Mischkammer wird zusätzlich ein kontinuierlicher Strom von 10 Teilen Di-n-butylphosphat (DBP) pro Stunde injiziert. In diese Mischkammer wurden nun 270 Teile Hexamethylendiamin (HDA) pro Stunde ebenfalls kontinuierlich eingespeist, wobei die Temperatur in der Mischkammer durch die Reaktionswärme auf 255 °C anstieg. Nach Verlassen der Mischkammer wurde das Produkt mit 2076 Teilen HDI einer Temperatur von 136 °C versetzt. Hierbei stellte sich eine Temperatur von 222 °C ein. Durch weitere externe Kühlung wurde die Mischung innerhalb von ca. 2 Minuten auf 180°C abgekühlt. Bei dieser Temperatur wurde noch 30 Minuten nachgetempert und die Rohware in einen Zwischenbehälter fließen lassen. Anschließend wurde aus der so erhaltene Rohware mit Hilfe der üblichen Dünnschicht-Destillationstechnik überschüssiges HDI abgetrennt. Es wurde ein als Lackhärter geeignetes Biuret-Polyisocyanat mit folgenden Kenndaten erhalten:

| | |
|---|---|
| NCO: | 22,0 % |
| Viskosität: | 9 500 mPas (23°C) |

### Beispiel 2

In einer Versuchsapparatur zur kontinuierlichen Herstellung von Biuret-Polyisocyanaten wurden 4600 Teile Hexamethylendiisocyanat (HDI) pro Stunde bei 214°C kontinuierlich durch eine Reaktionsmischkammer geleitet. In die HDI-Zuleitung kurz vor der Mischkammer wird zusätzlich ein kontinuierlicher Strom von 10 Teilen Di-n-butylphosphat (DBP) pro Stunde injiziert. In diese Mischkammer wurden nun 270 Teile Hexamethylendiamin (HDA) pro Stunde ebenfalls kontinuierlich eingespeist, wobei die Temperatur in der Mischkammer durch die Reaktionswärme auf 255 °C anstieg. Nach Verlassen der Mischkammer wurde das Produkt mit 2076 Teilen HDI mit einer Temperatur von 170°C versetzt und 90 Sekunden bei 230°C getempert. Danach wird durch externe Kühlung innerhalb von ca. 2 Minuten auf <180°C abgekühlt und in einen Zwischenbehälter überführt. Anschließend wurde aus der so erhaltene Rohware mit Hilfe der üblichen Dünnschicht-Destillationstechnik überschüssiges HDI abgetrennt. Es wurde ein als Lackhärter geeignetes Biuret-Polyisocyanat mit folgenden Kenndaten erhalten:

| | |
|---|---|
| NCO: | 22,1 % |
| Viskosität: | 9 300 mPas (23°C) |

### Vergleichsbeisviel 1

(Vergleich nach EP-B 1158013)

In einer Versuchsapparatur zur kontinuierlichen Herstellung von Biuret-Polyisocyanaten wurden 6670 Teile Hexamethylendiisocyanat (HDI) pro Stunde bei 250°C kontinuierlich durch eine Reaktionsmischkammer geleitet. In die HDI-Zuleitung kurz vor der Mischkammer wurde zusätzlich ein kontinuierlicher Strom von 10 Teilen Di-n-butylphosphat (DBP) pro Stunde injiziert. In diese Mischkammer wurden nun 270 Teile Hexamethylendiamin (HDA) pro Stunde ebenfalls kontinuierlich eingespeist, wobei die Temperatur in der Mischkammer durch die Reaktionswärme auf 280 °C anstieg. Nach Verlassen der Mischkammer wurde das Produkt innerhalb weniger Sekunden auf 180°C abgekühlt und bei 180 - 140°C noch einige Minuten nachgetempert. Anschließend wurde aus der so erhaltene Rohware mit Hilfe der üblichen Dünnschicht-Destillationstechnik überschüssiges HDI abgetrennt. Es wurde ein als Lackhärter geeignetes Biuret-Polyisocyanat mit folgenden Kenndaten erhalten:

| | |
|---|---|
| NCO: | 21,8 % |
| Viskosität: | 11 100 mPas (23°C) |

### Vereleichsbeispiel 2

(Vergleich nach EP-B 1158013)

Wie in Vergleichsbeispiel 1 beschrieben wurde der Mengenstrom HDI bei 230 °C kontinuierlich in die Mischkammer geleitet, mit DBP versetzt und HDA kontinuierlich eingespeist. Die Temperatur in der Mischkammer steigt auf ca. 260 °C an und man erhält nach Abkühlen, Nachtemperung und Aufarbeitung ein Biuretpolyisocyanat mit den folgenden Kenndaten:

| | |
|---|---|
| NCO: | 22,1 % |
| Viskosität: | 9 520 mPas (23°C) |

Wie Beispiele 1 und 2 und Vergleichsbeispiele 1 und 2 belegen müssen in den erfindungsgemäßen Beispielen nur jeweils 4600 Teile HDI pro Stunde auf 214 °C erhitzt werden, während in den Vergleichsbeispielen 6670 Teile HDI auf 250 °C oder zumindest 230 °C erhitzt werden müssen. Das erfindungsgemäße Verfahren erlaubt eine deutliche Energieeinsparung.

### Beispiel 3

In einer Versuchsapparatur zur kontinuierlichen Herstellung von Biuret-Polyisocyanaten wurden 6670 Teile Hexamethylendiisocyanat (HDI) pro Stunde bei 225°C kontinuierlich durch eine Reaktionsmischkammer geleitet. In die HDI-Zuleitung kurz vor der Mischkammer wurde zusätzlich ein kontinuierlicher Strom von 10 Teilen Di-n-butylphosphat (DBP) pro Stunde injiziert. In diese Mischkammer wurden nun 270 Teile Hexamethylendiamin (HDA) pro Stunde ebenfalls kontinuierlich eingespeist, wobei die Temperatur in der Mischkammer durch die Reaktionswärme auf 255 °C anstieg. Nach Verlassen der Mischkammer wurde das Produkt mit 5090 Teilen HDI mit einer Temperatur von 85 °C versetzt und damit die Mischung auf 180°C abgekühlt. Bei dieser Temperatur wurde noch 2 Stunden nachgetempert und die Rohware dann in einen Zwischenbehälter überführt. Anschließend wurde aus der so erhaltene Rohware mit Hilfe der üblichen Dünnschicht-Destillationstechnik überschüssiges HDI abgetrennt. Es wurde ein als Lackhärter geeignetes Biuret-Polyisocyanat mit folgenden Kenndaten erhalten:

| | |
|---|---|
| NCO: | 23,5 % |
| Viskosität: | 2 700 mPas (23°C) |

Dieses Beispiel 3 zeigt, dass bei Herstellung eines Biuretpolyisocyanats mit vergleichsweise niedriger Viskosität nur 6670 Teile (57 %) von in Summe 11760 Teilen HDI auf die hohe Temperatur von 225 °C erhitzt werden müssen. Gleichzeitig ist das HDI/HDA-Verhältnis der ersten Reaktionsstufe problemlos in der gleichen Apparatur mit gleichen Düsengrößen herstellbar wie die Beispiele 1 und 2 sowie die Vergleichsbeispiele.

Wie in EP-B 1158013 ausgeführt, wirken Zusätze von Säuren wie beispielsweise Dibutylphosphat reaktionsbeschleunigend, tragen zur Monomerenstabilität der Produkte bei und reduzieren die Empfindlichkeit der Produkte gegenüber feuchten Lösemitteln. Hierbei sind bei der Reaktion größere Mengen (0,05 - 0,5 %) gegenüber 0,02 - 0,5 % besonders bevorzugt (siehe Seite 3 und 4). In EP-B 1158013 werden in Bsp. 6a 0,15 Gew.-% DBP verwendet, in Bsp. 7a jedoch 0,25 Gew.-% DBP. Wie bereits dort beschrieben wirkt sich der höhere Säurezusatz besonders vorteilhaft aus, wie die monomeren HDI-Gehalte nach 1 Woche/80 °C-Lagerung belegen: 0,45 % HDI bei 0,25 Gew.-% DBP-Zusatz gegenüber 0,58 % HDI bei 0,15 Gew.-% Zusatz. Im erfindungsgemäßen Verfahren ist es nun möglich die DBP-Konzentration im ersten Reaktionsschritt a) zu maximieren, ohne die Gesamtmenge an DPB bezogen auf die im Verfahren insgesamt eingesetzte Menge an Ausgangsmaterial zu erhöhen. Man kann sich so die in EP-B 1158013 beschriebenen positive Auswirkungen zu Nutze machen, ohne über die 1,0 Gew.-%, vorzugsweise 0,5 Gew.-%, Säurezusatz hinausgehen zu müssen und ohne das Biuretpolyisocyanat zu sehr mit Folgeprodukten im Spurenbereich zu verunreinigen.

## Patentansprüche

1. Verfahren zur kontinuierlichen Herstellung von Polyisocyanaten mit Biuretstruktur durch kontinuierliche Umsetzung von überschüssigen Mengen an organischen Isocyanaten enthaltend ausschließlich aliphatisch und/oder cycloaliphatisch gebundene Isocyanatgruppen (A) mit organischen Aminen enthaltend ausschließlich aliphatisch und/oder cycloaliphatisch gebundene primäre Aminogruppen (B), indem man
a) eine erste Menge der Isocyanatkomponente A mit der Aminkomponente B vermischt, wobei die Temperatur oberhalb von 170 °C gehalten wird, und
b) eine zweite Menge der Isocyanatkomponente A mit einer Temperatur von 20 bis 250 °C dem Strom des aus Schritt a) resultierenden Reaktionsgemisches zudosiert,
wobei das Verhältnis der pro Zeiteinheit in Stufe a) zu b) eingespeisten Gewichtsteile der Isocyanatkomponente A 1:9 bis 9:1 beträgt, und die Anzahl der in a) und b) insgesamt pro Zeiteinheit eingespeisten NCO-Gruppen zur Zahl der pro Zeiteinheit eingespeisten NH₂-Gruppen mindestens 4:1 beträgt.

2. Verfahren zur kontinuierlichen Herstellung von Polyisocyanaten mit Biuretstruktur nach Anspruch 1, wobei als Isocyanatkomponente A HDI verwendet wird.

3. Verfahren zur kontinuierlichen Herstellung von Polyisocyanaten mit Biuretstruktur nach Anspruch 1 oder 2, wobei als Aminkomponente B HDA verwendet wird.

4. Verfahren zur kontinuierlichen Herstellung von Polyisocyanaten mit Biuretstruktur nach einem der Ansprüche 1 bis 3, wobei die Anzahl der in a) und b) insgesamt pro Zeiteinheit eingespeisten NCO-Gruppen zur Zahl der pro Zeiteinheit eingespeisten NH₂-Gruppen 18:1 bis 25:1 beträgt.

5. Verfahren zur kontinuierlichen Herstellung von Polyisocyanaten mit Biuretstruktur nach einem der Ansprüche 1 bis 4, wobei das Verhältnis der pro Zeiteinheit in Stufe a) zu b) eingespeisten Gewichtsteile der Isocyanatkomponente A 5:5 bis 7,5: 2,5 beträgt.

6. Verfahren zur kontinuierlichen Herstellung von Polyisocyanaten mit Biuretstruktur nach einem der Ansprüche 1 bis 5, wobei der erste Teilstrom der Isocyanatkomponente A, bevor er dem Aminstrom B zugeführt wird, auf Temperaturen von 200 bis 240°C vorgeheizt wird.

7. Verfahren zur kontinuierlichen Herstellung von Polyisocyanaten mit Biuretstruktur nach einem der Ansprüche 1 bis 6, wobei die Temperatur in Stufe a) bei 230-320°C gehalten wird.

8. Verfahren zur kontinuierlichen Herstellung von Polyisocyanaten mit Biuretstruktur nach einem der Ansprüche 1 bis 7, wobei der zweite Teilstrom der Isocyanatkomponente A, welcher in Stufe b) zugesetzt wird, Temperaturen aufweist, die unterhalb der Temperaturen des ersten Isocyanastromes aus a) liegen.

9. Verfahren zur kontinuierlichen Herstellung von Polyisocyanaten mit Biuretstruktur nach einem der Ansprüche 1 bis 8, wobei das Verfahren katalysiert durchgeführt wird.

10. Verfahren zur kontinuierlichen Herstellung von Polyisocyanaten mit Biuretstruktur nach Anspruch 9, wobei als Katalysator eine oder mehrere Säuren oder deren Gemische verwendet werden.

11. Verfahren zur kontinuierlichen Herstellung von Polyisocyanaten mit Biuretstruktur nach Anspruch 10, wobei der Katalysator Di-n-butylphosphat ist.

12. Verfahren zur kontinuierlichen Herstellung von Polyisocyanaten mit Biuretstruktur nach einem der Ansprüche 1 bis 11, wobei die erhaltene Polyisocyanatlösung durch Extraktion oder Dünnschichtdestillation von überschüssigem Diisocyanat auf Gehalte < 0,5 Gew.-% monomeres Diisocyanat befreit wird.

## Claims

1. Process for the continuous preparation of polyisocyanates with a biuret structure by continuous reaction of excess amounts of organic isocyanates containing exclusively aliphatically and/or cycloaliphatically bonded isocyanate groups (A) with organic amines containing exclusively aliphatically and/or cycloaliphatically bonded primary amino groups (B) by
a) mixing a first amount of the isocyanate component A with the amine component B, during which the temperature is kept above 170 °C, and
b) metering a second amount of the isocyanate component A with a temperature of from 20 to 250 °C into the stream of the reaction mixture resulting from step a), wherein the ratio of the parts by weight of the isocyanate component A fed per unit time into stage a) to b) is 1:9 to 9:1, and the number of NCO groups fed per unit time in total into a) and b) to the number of NH₂ groups fed in per unit time is at least 4:1.

2. Process for the continuous preparation of polyisocyanates with a biuret structure according to claim 1, wherein HDI is used as the isocyanate component A.

3. Process for the continuous preparation of polyisocyanates with a biuret structure according to claim 1 or 2, wherein HDA is used as the amine component B.

4. Process for the continuous preparation of polyisocyanates with a biuret structure according to one of claims 1 to 3, wherein the number of NCO groups fed per unit time in total into a) and b) to the number of NH₂ groups fed in per unit time is 18:1 to 25:1.

5. Process for the continuous preparation of polyisocyanates with a biuret structure according to one of claims 1 to 4, wherein the ratio of the parts by weight of the isocyanate component A fed per unit time into stage a) to b) is 5:5 to 7.5:2.5.

6. Process for the continuous preparation of polyisocyanates with a biuret structure according to one of claims 1 to 5, wherein the first part stream of the isocyanate component A is preheated to temperatures of from 200 to 240 °C before it is fed to the amine stream B.

7. Process for the continuous preparation of polyisocyanates with a biuret structure according to one of claims 1 to 6, wherein the temperature in stage a) is kept at 230 - 320 °C.

8. Process for the continuous preparation of polyisocyanates with a biuret structure according to one of claims 1 to 7, wherein the second part stream of the isocyanate component A, which is added in stage b), has temperatures which are below the temperatures of the first isocyanate stream from a).

9. Process for the continuous preparation of polyisocyanates with a biuret structure according to one of claims 1 to 8, wherein the process is carried out under catalysis.

10. Process for the continuous preparation of polyisocyanates with a biuret structure according to claim 9, wherein one or more acids or mixtures thereof are used as the catalyst.

11. Process for the continuous preparation of polyisocyanates with a biuret structure according to claim 10, wherein the catalyst is di-n-butyl phosphate.

12. Process for the continuous preparation of polyisocyanates with a biuret structure according to one of claims 1 to 11, wherein the polyisocyanate solution obtained is freed from excess diisocyanate down to contents of < 0.5 wt.% of monomeric diisocyanate by extraction or thin film distillation.

## Revendications

1. Procédé de fabrication continue de polyisocyanates dotés d'une structure de biuret par mise en réaction continue de quantités excessives d'isocyanates organiques contenant exclusivement des groupes isocyanate à liaison aliphatique et/ou cycloaliphatique (A) avec des amines organiques contenant exclusivement des groupes amino primaires (B) à liaison aliphatique et/ou cycloaliphatique, selon lequel
a) une première quantité du composant isocyanate A est mélangée avec le composant amine B, la température étant maintenue au-dessus de 170 °C, et
b) une seconde quantité du composant isocyanate A à une température de 20 à 250 °C est ajoutée au courant du mélange réactionnel résultant de l'étape a),
le rapport entre les parties en poids du composant isocyanate A introduites par unité de temps à l'étape a) et à l'étape b) étant de 1:9 à 9:1, et le nombre de groupes NCO introduits en a) et en b) au total par unité de temps par rapport au nombre de groupes NH₂ introduits par unité de temps étant d'au moins 4:1.

2. Procédé de fabrication continue de polyisocyanates dotés d'une structure de biuret selon la revendication 1, dans lequel l'HDI est utilisé en tant que composant isocyanate A.

3. Procédé de fabrication continue de polyisocyanates dotés d'une structure de biuret selon la revendication 1 ou 2, dans lequel l'HDA est utilisée en tant que composant amine B.

4. Procédé de fabrication continue de polyisocyanates dotés d'une structure de biuret selon l'une quelconque des revendications 1 à 3, dans lequel le nombre de groupes NCO introduits en a) et en b) au total par unité de temps par rapport au nombre de groupes NH₂ introduits par unité de temps est de 18:1 à 25:1.

5. Procédé de fabrication continue de polyisocyanates dotés d'une structure de biuret selon l'une quelconque des revendications 1 à 4, dans lequel le rapport entre les parties en poids du composant isocyanate A introduites par unité de temps à l'étape a) et à l'étape b) est de 5:5 à 7,5:2,5.

6. Procédé de fabrication continue de polyisocyanates dotés d'une structure de biuret selon l'une quelconque des revendications 1 à 5, dans lequel le premier courant partiel du composant isocyanate A est préchauffé à des températures de 200 à 240 °C avant son introduction dans le courant d'amine B.

7. Procédé de fabrication continue de polyisocyanates dotés d'une structure de biuret selon l'une quelconque des revendications 1 à 6, dans lequel la température à l'étape a) est maintenue de 230 à 320 °C.

8. Procédé de fabrication continue de polyisocyanates dotés d'une structure de biuret selon l'une quelconque des revendications 1 à 7, dans lequel le second courant partiel du composant isocyanate A, qui est ajouté à l'étape b), présente des températures inférieures aux températures du premier courant d'isocyanate de a).

9. Procédé de fabrication continue de polyisocyanates dotés d'une structure de biuret selon l'une quelconque des revendications 1 à 8, dans lequel le procédé est réalisé sous catalyse.

10. Procédé de fabrication continue de polyisocyanates dotés d'une structure de biuret selon la revendication 9, dans lequel un ou plusieurs acides ou leurs mélanges sont utilisés en tant que catalyseur.

11. Procédé de fabrication continue de polyisocyanates dotés d'une structure de biuret selon la revendication 10, dans lequel le catalyseur est le phosphate de di-n-butyle.

12. Procédé de fabrication continue de polyisocyanates dotés d'une structure de biuret selon l'une quelconque des revendications 1 à 11, dans lequel la solution de polyisocyanate obtenue est débarrassée par extraction ou distillation à couche mince du diisocyanate en excès à des teneurs < 0,5 % en poids de diisocyanate monomère.
